**Europäisches Patentamt**

(19) **European Patent Office**

**Office européen des brevets**

(11) Numéro de publication: **0 031 050**
**B1**

(12) **FASCICULE DE BREVET EUROPEEN**

(45) Date de publication du fascicule du brevet: **21.12.83**

(51) Int. Cl.³: **A 23 L 1/10, C 13 L 1/08**

(21) Numéro de dépôt: **80107447.7**

(22) Date de dépôt: **28.11.80**

(54) Procédé de fabrication d'un produit alimentaire amylacé pulvérulent facilement miscible à l'eau.

(30) Priorité: **19.12.79 CH 11246/79**

(43) Date de publication de la demande:
**01.07.81 Bulletin 81/26**

(45) Mention de la délivrance du brevet:
**21.12.83 Bulletin 83/51**

(84) Etats contractants désignés:
**AT BE DE FR IT NL SE**

(56) Documents cités:
**DE - A - 2 153 839**
**FR - A - 551 660**
**FR - A - 2 351 174**
**GB - A - 513 464**
**GB - A - 1 121 100**
**GB - A - 1 140 897**
**GB - A - 1 193 549**
**US - A - 3 337 414**

**DIE STARKE, vol. 17, no. 9, septembre 1965**
**W.G. KINGMA: "Erfahrungen bei der**
**kontinuierlichen Verflüssigung von Stärke mit**
**Säure und alpha-Amylase", pages 284-289**

(73) Titulaire: **SOCIETE DES PRODUITS NESTLE S.A.**
**Case postale 353**
**CH-1800 Vevey (CH)**

(72) Inventeur: **Gasser, Rupert Josef**
**Tournelle 29**
**CH-1350 Orbe (CH)**
Inventeur: **Badertscher, Ernest**
**Chemin de Champ Bornu 7**
**CH-1350 Orbe (CH)**

Courier Press, Leamington Spa, England.

## Procédé de fabrication d'un produit alimentaire amylacé pulvérulent facilement miscible à l'eau

La présente invention a pour objet un procédé de fabrication d'un produit alimentaire amylacé pulvérulent facilement miscible à l'eau, dans lequel on prépare un mélange d'une matière amylacée et d'eau, on liquéfie le mélange par hydrolyse enzymatique et on le sèche.

Des produits maltés obtenus à partir de matières premières riches en amidon telles que les céréales ou les pommes de terre par liquéfaction enzymatique de leur amidon existent depuis fort longtemps. Un procédé connu pour les fabriquer consiste à bouillir dans l'eau 20% en poids d'orge, de maïs, de riz, de blé ou de farine de sago, de tapioca ou de patate, chauffer la bouillie à la vapeur au-dessus du point d'ébullition pour gélifier l'amidon, ajouter du malt diastasique, brasser 2 h à 50°C, filtrer, concentrer la liqueur au 1/3 du volume initial et ajouter des glycérophosphates, des agents aromatisants, voire des solides lactiques ou des extraits de viande ou de poisson, la concentration pouvant être poursuivie jusqu'à la dessiccation. Ce procédé est coûteux du fait des énormes moyens à mettre en oeuvre pour la concentration et il entraîne une perte d'arôme importante lors de l'évaporation sous vide, en particulier une perte de goût de malt qui est partiellement volatil.

La production de dextrines ou de dextrose à partie de matières premières riches en amidon telles que les céréales ou les pommes de terre est également pratiquée depuis fort longtemps. Le procédé classique consiste à liquéfier tout d'abord une bouillie aqueuse de la matière première ou de son amidon par hydrolyse acide ou par hydrolyse enzymatique à l'aide d'une alphaamylase, puis à saccharifier la liqueur fluide en l'hydrolysant à l'aide d'une amyloglucosidase ou betaamylase. Un procédé récent de ce type consiste à réaliser l'hydrolyse à l'alphaamylase de l'amidon granulaire cru, non gélifié, à ajoiter progressivement de l'amidon granulaire cru à la bouillie en cours d'hydrolyse tout en élevant progressivement la température. Ce procédé permet d'obtenir des teneurs en matière sèche très élevées, sans jamais atteindre le stade de la gélification, donc en conservant une viscosité faible. La bouillie liquéfiée concentrée crue peut être utilisée ensuite telle quelle pour toute application usuelle des maltodextrines ou autres amidons liquéfiés, ou comme matière première pour une saccharification plus poussée. Quoique ce procédé permette l'économie de la concentration, il est extrêmement dangereux parce que tout agent contaminant présent dans la matière première peut se développer librement et il débouche sur la production de denrées à conservation très limitée. Dans un autre procédé connu de ce type, l'étape d'hydrolyse avec une alphaamylase est supprimée et l'on travaille dès le début avec une amyloglucosidase. Si le démarrage en est rendu plus lent, le taux d'hydrolyse final que l'on peut atteindre, exprimé en équivalent de dextrose ou glucose (DE), est plus élevé. Dans ce procédé, on chauffe à la vapeur à 140—150°C une pâte, à 30% de matière de sèche, d'amidon de maïs ou de pomme de terre, on la refroidit à 100°C par expansion, puis à 50—60°C dans un courant d'air, on la mélange avec une partie que l'on recycle de la pâte liquéfiée plus loin dans un convertisseur et on conduit le mélange en continu à ce convertisseur. La partie non recyclée de la pâte liquéfiée est conduite dans des convertisseurs discontinus où la saccharification se poursuit. On atteint ainsi des DE supérieurs à 99%. Ce procédé est difficile à contrôler, notamment au niveau du refroidissement et ne se prête pas un séchage par pulvérisation, le glucose n'étant séchable par pulvérisation qu'en solution diluée.

Enfin, dans le domaine précis de la présente invention, on connaît les déjeuners instantanés à base de céréales spécialement destinés aux enfants et présentés sous forme de flocons. Ceux-ci peuvent être fabriqués en préparant une pâte ou suspension épaisse d'une parine de riz, d'avoine, de blé, de maïs, d'orge ou d'un mélange de ces farines et en la séchant sur tambours. Il est connu qu'en choisissant des conditions de séchage appropriées on peut réaliser à la fois la gélification et l'hydrolyse enzymatique de l'amidon sur le tambour même, ce qui donne un produit digeste et facile à reconstituer. Cependant, ce procédé entraîne inévitablement une caramélisation et une maillardisation du produit, ce qui en modifie le goût et en diminue la valeur nutritive.

US—A—3337414 décrit un procédé dans lequel une petite quantité de dispersion d'amidon présentant une teneur en matière sèche de environ 30 à 34% est traitée quelques secondes à 130—160°C par injection de vapeur puis mélangée-diluée avec une grande quantité de dispersion présaccharifiée refroidie et recyclée, le mélange de dispersion d'amidon frais et de dispersion recyclée étant soumis à une présaccharification enzymatique et une petite quantité de dispersion présaccharifiée non recyclée étant conduite vers une étape finale de saccharification complète. Avec ce procédé il est possible de travailler à la limite des concentrations et des viscosités permettant encore à une dispersion aqueuse d'amidon d'être pompée et traitée avec des installations et appareils classiques. Ce document n'indique cependant pas comment on pourrait dépasser notablement cette limite située à environ 35% de matière sèche.

Le présent procédé doit pallier les lacunes et les inconvénients des procédés cités ci-dessus.

Le procédé selon la présente invention est caractérisé par le fait qu'on prépare un premier mélange de matière amylacée et d'eau, on le cuit et on le liquéfie par hydrolyse enzymatique, on prépare un second mélange de matière amylacée, d'eau et d'une partie au moins du premier mélange liquéfié, de manière que le second mélange présente une teneur en matière sèche de 35 à 45% en poids, on cuit le second mélange, on le liquéfie par hydrolyse enzymatique et on en sèche une partie au moins par pulvérisation.

De préférence, on réalise le présent procédé en continu, une partie du mélange liquéfié étant recyclée au niveau du mélange de la matière amylacée et de l'eau.

Le présent procédé permet d'éviter les installations et les opérations coûteuses de la concentration, tout en évitant toute réaction du genre maillardisation ou caramélisation. Il permet d'obtenir un produit pulvérulent qui présente des qualités tout à fait extraordinaires de miscibilité, de fluidité et de solubilité à froid et à chaud, un arôme intact d'une pureté incomparable et une absence pratiquement totale de rétrogradation de l'amidon à la conservation, autrement dit d'excellentes qualités de conservation.

Pour mettre en oeuvre le présent procédé, on peut utiliser une matière amylacée telle qu'un gruau, une farine, voire même un amidon de céréale ou de mélange de céréales, notamment de blé, d'orge, d'avoine, de seigle, de riz et/ou de maïs. De préférence, on utilise un produit de meunerie de tout degré d'extraction, de la farine fleur (60% du grain) au gruau (95% du grain). On peut obtenir un produit présentant un goût particulièrement agréable en utilisant une composition de farine ou de gruau comprenant 80% en poids de blé, 10% de seigle et 10% d'avoine. En outre, on peut utiliser également d'autres matières végétales riches en amidon telles que les graines de légumineuses et certaines tubercules comme les pommes de terre ou certaines racines comme le céleri.

De préférence, on confère au premier mélange une teneur en matrière séche de 25 à 35% en poids.

Pour cuire le mélange, on peut le porter par injection de vapeur à une température de 130 à 160°C par exemple et le tenir à cette température durant 15 à 60 s. On peut également le porter à des températures inférieures par des moyens adéquats et l'y tenir plus longtemps. Etant donné la viscosité du mélange stérilisé et gélifié par la cuisson, on peut prévoir un mélangeur statique sur la ligne, par exemple juste après la buse d'unjection de vapeur.

Après la cuisson, on peut refroidir le mélange directement à la température d'hydrolyse, par détente ou à l'aide d'un échangeur de chaleur, selon les cas. Lorsqu'on effectue la cuisson par injection de vapeur, on préfère réaliser le refroidissement par détente sous vide, et ceci directement dans une enceinte d'hydrolyse.

On effectue de préférence l'hydrolyse à une température de 50 à 90°C, avec du malt diastasique. En lieu et place du malt, on peut également utiliser une combinaison d'alpha- et betaamylases du commerce, d'origine fongique ou bactérielle par exemple. En utilisant une combinaison d'enzymes telle que celle présente dans un extrait de malt diastasique, on peut jouer sur les deux tableaux de la fluidité et du pouvoir sucrant avec une grande souplesse, selon l'usage auquel on destine le produit final. Si par exemple, on désire une poudre à pouvoir épaississant pour les potages, on peut favoriser l'activité de l'alphaamylase qui fragmente l'amidon au hasard en courtes chaînes, autrement dit en dextrines. Si au contraire, on désire un poudre à fort pouvoir sucrant pour une boisson par exemple, on peut favoriser l'activité de la betaamylase qui détache des molécules de maltose l'une après l'autre, à partir d'une extrémité de l'amidon. Dans le premier cas, on peut réaliser l'hydrolyse en un temps relativement court, de l'ordre de 10 min, à une température relativement élevée, de l'ordre de 80°C, mais sans dépasser 90°C, ce qui inactiverait les enzymes. Dans le second cas, on peut réaliser l'hydrolyse en un temps relativement long, de l'ordre de 60 min, à une température relativement basse, de l'ordre de 60°C, mais sans descendre au-dessous de 50°C ce qui ralentirait trop l'action des enzymes. Au cas où l'on désire produire une poudre pour un petit déjeuner instantané, on préfère choisir des conditions intermédiaires, à savoir une température d'environ 65—70°C et une durée de 30 à 45 min. La température d'hydrolyse peut être contrôlée de manière très précise en refroidissant le mélange cuit, autrement dit la bouillie de céréale cuite, par détente dans une enceinte d'hydrolyse sous un vide correspondant à la pression de vapeur du mélange à la température choisie. Si la durée d'hydrolyse choisie excède le temps de séjour possible dans cette enceinte, la bouillie en cours d'hydrolyse peut être transféréee dans une ou successivement dans plusieurs cuves d'attente à pression atmosphérique où l'hydrolyse peut se poursuivre à la même température. Quant à la quantité d'enzymes à utiliser, elle dépend entre autres de l'activité de l'enzyme, de sa composition et des conditions d'hydrolyse. A titre d'orientation, on peut mentionner un chiffre de l'ordre de 5% d'extrait de malt diastasique par rapport au poids de farine ou de gruau mis en oeuvre pour la production d'un petit déjeuner instantané.

Le procédé peut donc être mis en oeuvre en continu, avec recyclage, ou en discontinu, en deux temps. La partie du mélange liquéfié qui n'est pas recyclée, dans le premier cas, ou le second mélange liquéfié, dans le second case, peut être stérilisée, par injection de vapaur à 115—150°C durant 5—20 s par exemple, avant d'être séchée par pulvérisation. Avant de stériliser ce mélange liquéfié, que l'on pourrait nommer liqueur de céréale, on peut lui ajouter des compléments nutritifs, notamment des solides du lait écrémé ou du petit-lait en poudre ou des matières grasses, par exemple, selon l'usage auquel on destine le produit final. On peut également ajouter certains de ces compléments avant l'hydrolyse et même avant la cuisson. Si l'on désire produire une poudre pour une soupe par exemple, on ajoutera de préférence de la matière grasse, notamment une graisse végétale telle qu'une graisse de palme, de coco, de tournesol, de maïs et/ou de soja, à raison par exemple de 20 à 40% en poids de la farine ou du gruau mis en oeuvre. On obtient dans ce cas une poudre grasse dans laquelle la graisse est si bien liée qu'elle ne se sépare pas et ne forme pas d'yeux lorsqu'on dissout la poudre dans l'eau. Cela signifie aussi que les arômes pris dans la graisse y demeurent, ce qui confère au potage une qualité organoleptique incomparable. Enfin, si l'on désire produire une poudre pour boissons acides, on peut ajouter des compléments nutritifs au mélange liquéfié, notamment, du lactose, avant de le stériliser, et le soumettre à une fermentation, notamment par des bactéries lactiques, après l'avoir stérilisé et avant de le sécher par pulverisation.

Les exemples qui suivent sont présentés à titre d'illustration de la présente invention. Les pourcentages y sont donnés en poids.

Exemple 1

Dans un processus en continu, on mélange 200 kg/h d'une composition à 14% d'humidité comprenant 80% de farine de blé, 10% de farine de seigle et 10% de farine d'avoine avec 184 kg/h d'eau potable à 12°C et 426 kg/h de liqueur de céréale recyclée présentant une teneur en matière sèche de 40,4%. On chauffe le mélange à 135°C par injection de vapeur, on le fait passer dans un mélangeur statique, on le fait suivre dans une conduite d'attente et, après l'avoir maintenu ainsi à 135°C durant un temps total de 30 s, on le refroidit à 70°C par détente dans une cuve étanche à double paroi et mélangeur où une pression égale à la pression de vapeur du mélange à 70°C est maintenue à l'aide d'un condenseur. On déverse dans cette même cuve 10 kg/h d'extrait de malt diastasique dilué dans 20 kg/h d'eau potable. Le mélange doucement agité séjourne 15 min dans cette première cuve, puis parcourt un circuit de 3 cuves à paroi double et mélangeur où l'on maintient une température de 70°C à pression atmosphérique, le temps de séjour dans chaque cuve étant de 10 min, ce qui donne une durée totale d'hydrolyse de 45 min.

On divise le flot de liqueur sortant de la dernière cuve en deux courants, l'un de 426 kg/h que l'on recycle et l'autre de 449 kg/h, à 40,4% de matière sèche que l'on dirige sur la ligne de séchage. On mélange intimement à ce dernier 52 kg/h de graisse végétale contenant 25% d'acide linoléique, on stérilise le tout par injection de vapeur à 120°C durant 20 s, on le refroidit à 70°C par détente à pression atmosphérique et on le séche par pulvérisation.

On obtient une poudre ocre clair très fluide, agréablement odorante, qui se dissout très bien dans l'eau aussi bien froide que chaude et ne forme pas d'yeux. Cette poudre ne présente aucune rétrogradation de l'amidon après plusieurs mois de conservation en boîte métallique, sous atmosphère inerte et à température ambiante. Elle constitue une base de première qualité pour la préparation de boissons instantanées pour le petit déjeuner. A cet effet, on peut par exemple mélanger 786 g de cette base, 14 g de sel et épices et 200 g de poudre de fruits, notamment de poires, et utiliser ce mélange à raison de 35 g dilués dans 125 cc de lait froid.

Exemple 2

Dans un processus semblable à celui de l'exemple 1 mais dans lequel on n'utilise que la première cuve, on hydrolyse soit du blé, soit des pommes de terre (lavées, pelées et broyées), soit des racines de céleri (parées et broyées), soit un mélange de pommes de terre et céleri, on effectue l'hydrolyse à 80—85°C durant 10 min, on ajoute ou l'on n'ajoute pas environ 10% de graisse et/ou environ 10% de poudre de lait écrémé avant stérilisation par injection de vapeur et séchange par pulvérisation, et l'on obtient à chaque fois une base excellente pour la préparation de potages. 150 à 170 g de ces poudres additionnées de 10 g d'épices et 14 g de sel par exemple se prêtent à la préparation de 1,5 à 1,7 de potage.

Exemple 3

Dans un processus semblable à celui décrit à l'exemple 1, on réalise l'hydrolyse à 64°C à l'aide de 8,15 kg/h d'extrait de malt diastasique dilués dans 16,3 kg/h d'eau et l'on sort 985 kg/h de liqueur à 35,4% de matière sèche de la dernière cuve. On en recycle 396 kg/h que l'on mélange à 163 kg/h de farine, 30 kg/h de poudre de lait écrémé et 258 kg/h d'eau au début du cycle. On dirige les 499 kg/h restant sur la ligne de séchage. On mélange intimement à cette liqueur 25 kg/h de graisse végétale. On stérilise la liqueur par injection de vapeur à 120°C durant 5 s, on la refroidit à 70°C par détente à pression atmosphérique puis à 40°C dans un échangeur de chaleur tubulaire et on la conduit dans une cuve de fermentation où on l'acidified biologiquement. A cet effet, on l'inocule avec 2% d'une culture classique de yogourt composée de *Lactobacillus bulgaricus* et de *Streptococcus thermophilus* et on laisse fermenter durant 4 h à 40°C. La liqueur fermentée est alors séchée par pulvérisation.

On obtient une poudre couleur crème qui s'écoule très bien, qui a une flaveur agréable et qui se dissout très bien dans l'eau même froide. Elle se conserve bien et elle constitue une base de première qualité pour la préparation de boissons nutritives. A cet effect, on peut mélanger 70% de cette poudre acidifiée, 15% de flocons obtenus par séchage sur tambour de la même liqueur non acidifiée et 15% de poudre de fruits, par exemple, et utiliser ce mélange à raison de 35 g dilués dans 125 cc de lait froid. Cela donne une boisson aux fruits complète ayant une consistance assez épaisse rappelant celle du yogourt brassé.

D'autres possibilités typiques d'utilisation consistent par exemple à diluer dans 180 g d'eau 60 g de poudre acidifiée et aromatisée à l'orange avant séchage, ou à diluer 30 g de poudre acidifiée dans 200 cc de jus d'orange. Il faut noter que même dans ce dernier cas la boisson obtenue est parfaitement stable et ne présente aucun phénomène de séparation ou de sédimentation.

Exemple 4

Dans un processus semblable à celui de l'exemple 1, on réalise l'hydrolyse à 64°C durant 45 min et le flot de liqueur sortant de la dernière cuve présente une teneur en matière sèche de 41,1%. On divise ce flot en deux courants, l'une de 419 kg/h que l'on recycle et l'autre de 442 kg/h.

On conduit ce dernier dans une cuve d'attente où on le laisse reposer encore 3 h à 64°C, on le stérilise ensuite à 120°C par injection de vapeur, on le refroidit à 70°C par détente et on le sèche par pulvérisation.

On obtient une poudre à grand pouvoir sucrant qui se prête particulièrement bien à la préparation de boissons nutritives dans la composition desquelles on peut supprimer le saccharose.

Exemple 5

Dans un processus semblable à celui décrit à l'exemple 1, on réalise l'hydrolyse dans une seule cuve à 85°C durant 10 min. Le flot sortant de cette cuve présente une teneur en matière sèche de 40,6%. On le divise en deux courants, l'un de 424 kg/h que l'on recycle et l'autre de 448 kg/h que l'on stérilise 5 s à 120°C, refroidit à 70°C et séche par pulvèrisation.

On obtient une poudre à faible pouvoir sucrant et forte viscosité aprés reconstitution qui constitue une base idéale pour un potage instantané.

Exemple 6

Sur la ligne utilisée pour réaliser les fabrications décrites aux exemples précédents, on prépare de manière analogue des poudres instatanées à partie successivement de riz, de blé entier, de maïs jaune, de maïs blanc, de seigle, d'orge, d'avoine et d'amidon de maïs. Tous ces essais sont concluants. Le procédé reste la même. Seule la teneur en matière sèche ou la quantité recyclée peut varier dans le cas où la viscosité peur poser un problème à la cuisson, en particulier dans le case de l'orge.

Exemple 7

Sur la ligne utilisée pour réaliser les fabrications décrites aux exemples précédents, on effectue des essais avec deux enzymes bactériennes du commerce, une amylase et une alphaamylase, à partie d'amidon de maïs et de fécule de pommes de terre. On obtient des poudres qui présentent un goût parfaitement neutre.

Exemple 8

Dans un processus en discontinu, dans un premier temps, on mélange 100 kg de farine de blé à 14% d'humidité avec 200 kg d'eau potable à 50°C.

On chauffe le mélange à 135°C par injection de vapeur, on le fait passer dans un mélangeur statique, on le fait suivre dans une conduite d'attente et, après l'avoir maintenu ainsi durant un temps total de 30 s, on le refroidit à 64°C par détente dans une cuve étanche à double paroi et mélangeur où une pression égale à la pression de vapeur du mélange à 64°C est maintenue à l'aide d'un condenseur.

On déverse dans cette cuve 5 kg d'extrait de malt diastasique dilué dans 10 kg d'eau potable. Le mélange agité séjourne 15 min dans cette première cuve, puis est stocké durant 30 minutes dans une deuxième cuve à double paroi où la température est maintenue à 64°C à pression atmosphérique.

Dans un deuxième temps, on mélange 100 kg de farine de blé à 14% d'humidité avec la liqueur provenant de la première hydrolyse.

On chauffe ce deuxième mélange présentant une teneur en matière sèche de 41,7% à 135° par injection de vapeur de la même façon que lors du 1er chauffage.

Le refroidissement à 64°C s'effectue également par détente dans une cuve étanche dans laquelle on ajoute encore 5 kg d'extrait de malt diastasique dilué dans 10 kg d'eau potable.

Après 15 min d'attente sous agitation constante, le mélange est transféré dans une cuve à double manteau afin de maintenir le tout à 64°C durant 30 min.

La liqueur sortant de cette 2 ème cuve est stérilisée par injection de vapeur à 120°C durant 20 s. On refroidit par détente à pression atmosphérique et on sèche par pulvérisation cette liqueur à 40,6% de matière sèche.

On obtient une poudre semblable à celle décrite dans l'exemple 1.

En augmentant la durée d'hydrolyse dans la deuxième curve, on obtient également un poudre semblable à celle mentionnée à l'exemple 4.

On réalise les opérations des premier et deuxième temps ci-dessus soit à l'aide d'une seule et même installation, soit à l'aide de deux lignes identiques installées en série.

**Revendications**

1. Procédé de fabrication d'un produit alimentaire amylacé pulvérulent facilement miscible à l'eau, dans lequel on prépare un mélange d'une matière amylacée et d'eau, on liquéfie le mélange par hydrolyse enzymatique et on le sèche, caractérisé par le fait qu'on prépare un premier mélange de matière amylacée et d'eau, on le cuit et on le liquéfie par hydrolyse enzymatique, on prépare un second mélange de matière amylacée, d'eau et d'une partie au moins du premier mélange liquéfié, de manière que le second mélange présente une teneur en matière sèche de 35 à 45% en poids, on cuit le second mélange, on le liquéfie par hydrolyse enzymatique et on en sèche une partie au moins par pulvérisation.

2. Procédé selon la revendication 1, caractérisé par le fait qu'on le réalise en continu, une partie du mélange liquéfié étant recyclée au niveau du mélange de la matière amylacée et de l'eau.

3. Procédé selon la revendication 1, caractérisé par le fait que la matière amylacée est un gruau, une farine ou un amidon de céréale ou de mélange de céréales, notamment de blé, d'orge, d'avoine, de seigle, de riz et/ou de maïs.

4. Procédé selon la revendication 1, caractérisé par le fait que la matière amylacée se compose de gruau ou de farine de blé, de seigle et d'avoine, dans la proportion pondérale 80:10:10.

5. Procédé selon la revendication 1, caractérisé par le fait que le premier mélange présente une teneur en matière sèche de 25 à 35% en poids.

6. Procédé selon la revendication 1, caractérisé par le fait que pour cuire le mélange on le porte par injection de vapeur à une température de 130 à 160°C et on le tient à cette température durant 15 à 60 s.

7. Procédé selon la revendication 1, caractérisé par le fait qu'après la cuisson on refroidit le mélange à la température d'hydrolyse par détente dans une enceinte d'hydrolyse.

8. Procédé selon la revendication 1, caractérisé par le fait que l'on effectue l'hydrolyse à l'aide de malt diastasique à une température de 50 à 90°C.

9. Procédé selon la revendication 1, caractérisé par le fait qu'une partie au moins du second mélange liquéfié est stérilisée par injection de vapeur avant d'être séchée par pulvérisation.

10. Procédé selon la revendication 9, caractérisé par le fait que l'on ajoute au mélange liquéfié des compléments nutritifs, notamment des solides du lait écrémé ou du petit-lait ou des matières grasses, avant de le stériliser.

11. Procédé selon la revendication 9, caractérisé par le fait que l'on ajoute des compléments nutritifs au mélange, notamment du lactose, avant de le stériliser, et on le soumet à une fermentation, notamment par des bactéries lactiques, après l'avoir stérilisé et avant de le sécher par pulvérisation.

12. Procédé selon la revendication 9, caractérisé par le fait que l'on ajoute du lait écrémé en poudre au mélange avant l'hydrolyse ou avant la cuisson.

**Patentansprüche**

1. Verfahren zur Herstellung eines Pulverförmigen, leicht mit Wasser mischbaren, stärkehaltigen Nahrungsmittels, bei welchem ein Gemisch eines stärkehaltigen Materials mit Wasser hergestellt und das Gemisch durch enzymatische Hydrolyse verflüssigt und getrocknet wird, dadurch gekennzeichnet, daß man ein erstes Gemisch aus stärkehaltigem Material und Wasser herstellt, dieses Gemisch kocht und durch enzymatische Hydrolyse verflüssigt, daß man ein zweites Gemisch aus stärkehaltigen Material, Wasser und wenigstens einem Teil des ersten verflüssigten Gemisches herstellt, und zwar auf solche Art und Weise, daß das zweite Gemisch einen Gehalt an Trockenstoffen von 35 bis 45 Gew.-% aufweist, daß man das zweite Gemisch kocht, dasselbe durch enzymatische Hydrolyse verflüssigt und daß man wenigstens einen Teil dieses Gemisches durch Sprühtrocknen trocknet.

2. Verfahren nach Anspruch 1, dadurch gekennzeichnet, daß man das Verfahren Kontinuierlich ausführt, wobei ein Teil des verflüssigten Gemisches im Kreislauf auf das Niveau des Gemisches aus stärkehaltigem Material und Wasser zurückgeführt wird.

3. Verfahren nach Anspruch 1, dadurch gekennzeichnet, daß das stärkehaltige Material eine Grütze, ein Mehl oder eine Stärke von Getreide oder von einer Mischung von getreidearten ist, insbesondere von Weizen, Gerste, Hafer, Roggen, Reis und/oder Mais.

4. Verfahren nach Anspruch 1, dadurch gekennzeichnet, daß das stärkehaltige Material zusammengesetzt ist aus Grütze oder Mehl von Weizen, Roggen und Hafer im Gewichtsverhältnis von 80:10:10.

5. Verfahren nach Anspruch 1, dadurch gekennzeichnet, daß das erste Gemisch einen Gehalt an Trockenstoffen von 25 bis 35 Gew.-% aufweist.

6. Verfahren nach Anspruch 1, dadurch gekennzeichnet, daß man das Gemisch zum Kochen desselben durch Einspritzen von Dampf auf eine Temperatur von 130 bis 160°C bringt und daß man es während 15 bis 60 s (Sekunden) auf dieser Temperatur hält.

7. Verfahren nach Anspruch 1, dadurch gekennzeichnet, daß man das Gemisch nach dem Kochen durch Entspannen in einem Hydrolysenraum auf die Hydrolysentemperature abkühlt.

8. Verfahren nach Anspruch 1, dadurch gekennzeichnet, daß man die Hydrolyse mit Hilfe von Malzdiastase bei einer Temperatur von 5 bis 90°C ausführt.

9. Verfahren nach Anspruch 1, dadurch gekennzeichnet, daß wenigstens eine Teil des zweiten verflüssigten Gemisches vor dem Sprühtrocknen durch Einspritzen von Dampf sterilisiert wird.

10. Verfahren nach Anspruch 9, dadurch gekennzeichnet, daß man dem verflüssigten Gemisch Ernährungskomplemente, insbesondere Feststoffe von Magermilch oder von Molke oder Fettstoffe zusetzt, bevor man das Gemisch sterilisiert.

11. Verfahren nach Anspruch 9, dadurch gekennzeichnet, daß man dem Gemisch Ernährungskomplemente, insbesondere Laktose, zusetzt, bevor es sterilisiert wird, und daß man das Gemisch einer Fermentation, insbesondere durch Milchsäurebaktieren, unterwirft, nachdem das Gemisch sterilisiert worden ist und bevor es durch Sprühtrocknen getrocknet wird.

12. Verfahren nach Anspruch 9, dadurch gekennzeichnet, daß man dem Gemisch vor der Hydrolyse oder vor dem Kochen Magermilchpulver zusetzt.

**Claims**

1. A process for the production of a readily water-miscible powder-form amylaceous food product, which comprises preparing a mixture of amylaceous material and water, liquefying the mixture by enzymatic hydrolysis and drying it, characterized by preparing a first mixture of amylaceous material and water, cooking the mixture and liquefying it by enzymatic hydrolysis, preparing a second mixture of amylaceous material, water and at least part of the first liquefied mixture, so that the second mixture has a dry matter content of from 35 to 45% by weight, cooking the mixture, liquefying it by enzymatic hydrolysis and spray drying at least part thereof.

2. A process as claimed in Claim 1, which is carried out continuously, part of the liquefied mixture being recycled to the stage of mixing of amylaceous material and water.

3. A process as claimed in Claim 1, wherein the amylaceous material is a meal, a flour or a starch of a cereal or a mixture of cereals, especially of wheat, barley, oats, rye, rice and/or maize.

4. A process as claimed in Claim 1, wherein the amylaceous material consists of meal or flour of wheat, rye and oats in a ratio by weight of 80:10:10.

5. A process as claimed in Claim 1, wherein the first mixture has a dry matter content of from 25 to 35% by weight.

6. A process as claimed in Claim 1, wherein the mixture is cooked by the injection of steam to a temperature of from 130 to 160°C and kept at that temperature for 15 to 60 s.

7. A process as claimed in Claim 1, wherein after cooking, the mixture is cooled to the hydrolysis temperature by expansion in a hydrolysis vat.

8. A process as claimed in Claim 1, wherein hydrolysis is carried out with melt diastase at a temperature in the range from 50 to 90°C.

9. A process as claimed in Claim 1, wherein at least part of the liquefied second mixture is sterilised by the injection of steam before being spray dried.

10. A process as claimed in Claim 9, wherein nutritious complements are added to the liquefied mixture, especially solids of skim milk or whey or fats, before it is sterilised.

11. A process as claimed in Claim 9, wherein nutritious complements, especially lactose, are added to the mixture before it is sterilised and the mixture is subjected to fermentation, especially by lactic bacteria, after sterilisation and before spray drying.

12. A process as claimed in Claim 9, wherein skim milk powder is added to the mixture before hydrolysis or before cooking.